(19) Europäisches Patentamt
European Patent Office
Office européen des brevets

(11) Publication number: 0 381 532 A1

# (12) EUROPEAN PATENT APPLICATION

(21) Application number: 90301145.0

(51) Int. Cl.[5]: C07D 477/00, A61K 31/40

(22) Date of filing: 02.02.90

(30) Priority: 02.02.89 US 305999

(43) Date of publication of application:
08.08.90 Bulletin 90/32

(84) Designated Contracting States:
CH DE FR GB IT LI NL

(71) Applicant: MERCK & CO. INC.
126, East Lincoln Avenue P.O. Box 2000
Rahway New Jersey 07065-0900(US)

(72) Inventor: Greenlee, Mark L.
1470 Campbell St., PB No. 1
Rahway, New Jersey 07065(US)
Inventor: Dininno, Frank P.
5 Benjamin Court
Old Bridge, New Jersey 08857(US)
Inventor: Salzmann, Thomas N.
154 Meadowbrook Drive
No. Plainfield, New Jersey 07062(US)

(74) Representative: Hesketh, Alan, Dr. et al
European Patent Department Merck & Co., Inc. Terlings Park Eastwick Road
Harlow Essex, CM20 2QR(GB)

## (54) Novel 6-amido-1-methyl carbapenams.

(57) New antibacterial 6-amido-1-methyl-carbapenams and a process for their synthesis through new intermediates from carbapenems are disclosed.



Xerox Copy Centre

# NOVEL 6-AMIDO-1-METHYL-CARBAPENAMS

## BACKGROUND OF THE INVENTION

### 1) Field of the Invention

This invention relates to new antibacterial 6-amido-1-methyl-carbapenams and a process for their synthesis involving new intermediates.

### 2) Brief Description of Disclosures in the Art

Carbapenem antibiotics, for example imipenem (see U.S Patent Nos. 3,950,357 and 4,194,047), are well known for treating a broad spectrum of gram-negative and gram-positive bacterial infections.

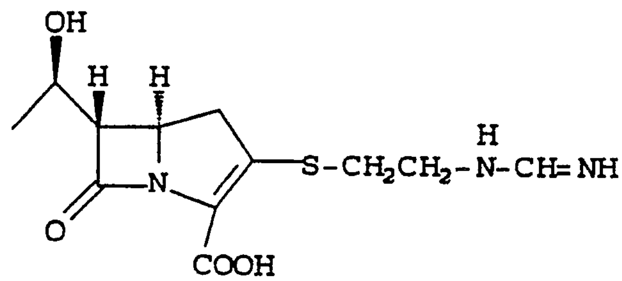

Imipenem

Although possibly not as well known as imipenem type carbapenem antibiotics, other carbapenems and related carbapenams have been described and reported to have an antibacterial effect. Relevantly, these other compounds include the 1-β-methylcarbapenems, the 6-amidocarbapenems and the 6-amidocarbapenams.

1-β-Methylcarbapenems, as described in the reference Heterocycles, 1984, Vol. 21, pp. 29-40 by D.H. Shih, F. Baker, L. Cama and B.G. Christensen, are extremely useful and effective broad spectrum antibiotics. For example, these compounds are useful against a wide variety of bacteria including gram-positive bacteria such as S. aureus, Strep. sp., B. subtilis, and gram-negative bacteria such as E. coli, Shigella sp., Enterobacter sp., Klebsiella sp., Proteus, Serratia and Pseudomonas sp.

6-amidocarbapenems and 6-amidocarbapenams are known in the art. 1-H-6-amidocarbapenems and 1-methyl-6-aminocarbapenems are disclosed in U.S. Pat. Nos. 4,260,627; 4,206,219; 4,217,453; 4,218,459; 4,218,463; 4,277,482; and 4,298,741. 1-hydroxy, 1-acetoxy or 1,1-oxo-carbapenems with 6-position amido side chains are disclosed in EPO Publication No. 040,494 and U.S. Pat. No. 4,348,264 to Pfizer. 6-phthalimido-2-thio-carbapenems are disclosed in Japanese KoKai 58-174,382. 1-alkyl-1-alkoxycarbonyl, cyano, or COR-substituted-6-amidocarbapenems are disclosed in EPO Publication No. 045,198 to Takeda Chem. Ind. Ltd. 6-aminocarbapenams are disclosed in EPO, 237,027 to Sanraku.

U.S. Pat. No. 4,347,355 and BE 887,618, to Abbott disclose 1,1-dialkyl-6-amidocarbapenams and 1,1-dialkyl-6-amidocarbapenems. The compounds are reported to be effective antibacterials.

U.S. Pat. No. 4,407,815 and EPO Pub. Nos. 634,443 and 073,100 to Pearson, et al., disclose 6-amidocarbapenams and 6-amidocarbapenems. As above, those compounds are effective antibacterials.

New antibacterial compounds are constantly being searched for to enhance the potency and decrease the side effects of current existing carbapenem antibiotics. Thus far, 1-methyl-6-amidocarbapenams have not been disclosed in the art.

## SUMMARY OF THE INVENTION

It has been found that a new class of compounds, 6-amido-1-methyl-carbapenams exhibit antibacterial activity and can be synthesized from 6-amido-1-methyl-carbapenem starting materials through new intermediates.

By this invention, there are provided compounds of the structural formula:

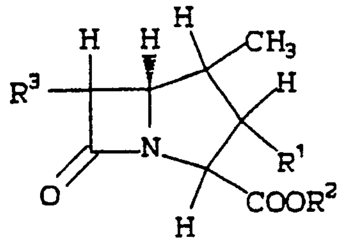

(I)

wherein
$R^1$ is -H or -S-$R^{1a}$;
$R^{1a}$ is $-CH_3$, $-C_6H_5$ or $-(CH_2)_nCN$;
n is 1 to 6;
$R^2$ is hydrogen, protecting group, pharmaceutically acceptable salt, or biolabile ester group;

$R^3$ is;

(a)

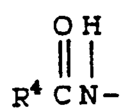

or

(b)

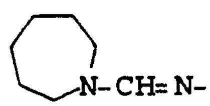

; and

$R^4$ is (a) allyloxy;

(b) 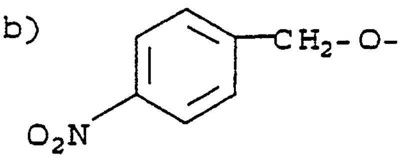 (c) 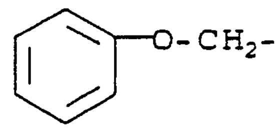

(d) 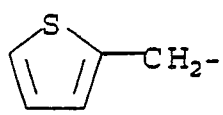 or (e) 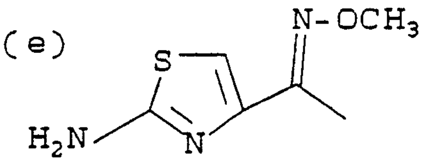

DETAILED DESCRIPTION OF THE INVENTION

The invention can be readily understood by reference to the following flow sheets which exhibit the processes for the synthesis of the instantly claimed compounds which are the preferred embodiments.

## Flow Sheet A (part I)

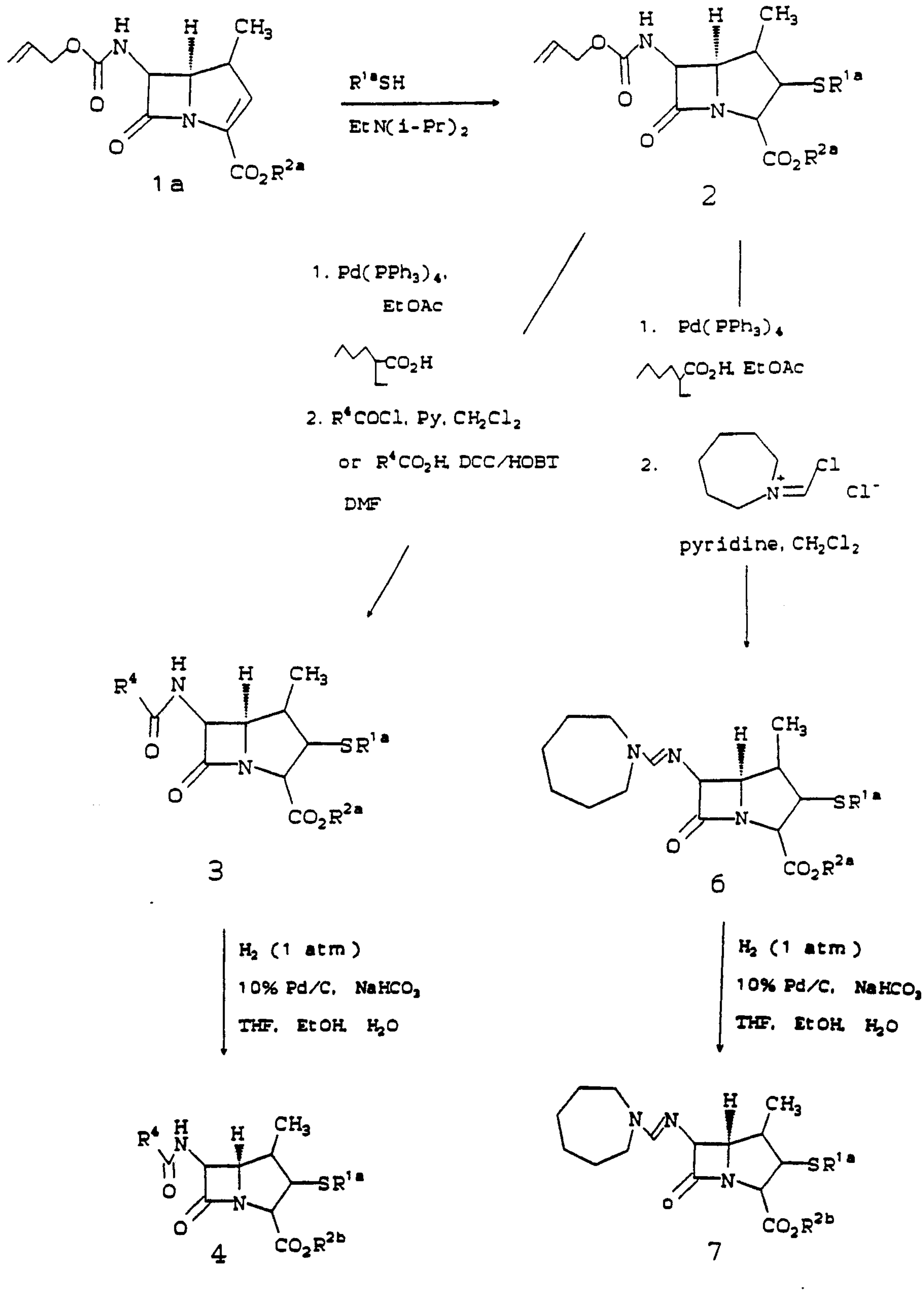

Flow Sheet A (part II)

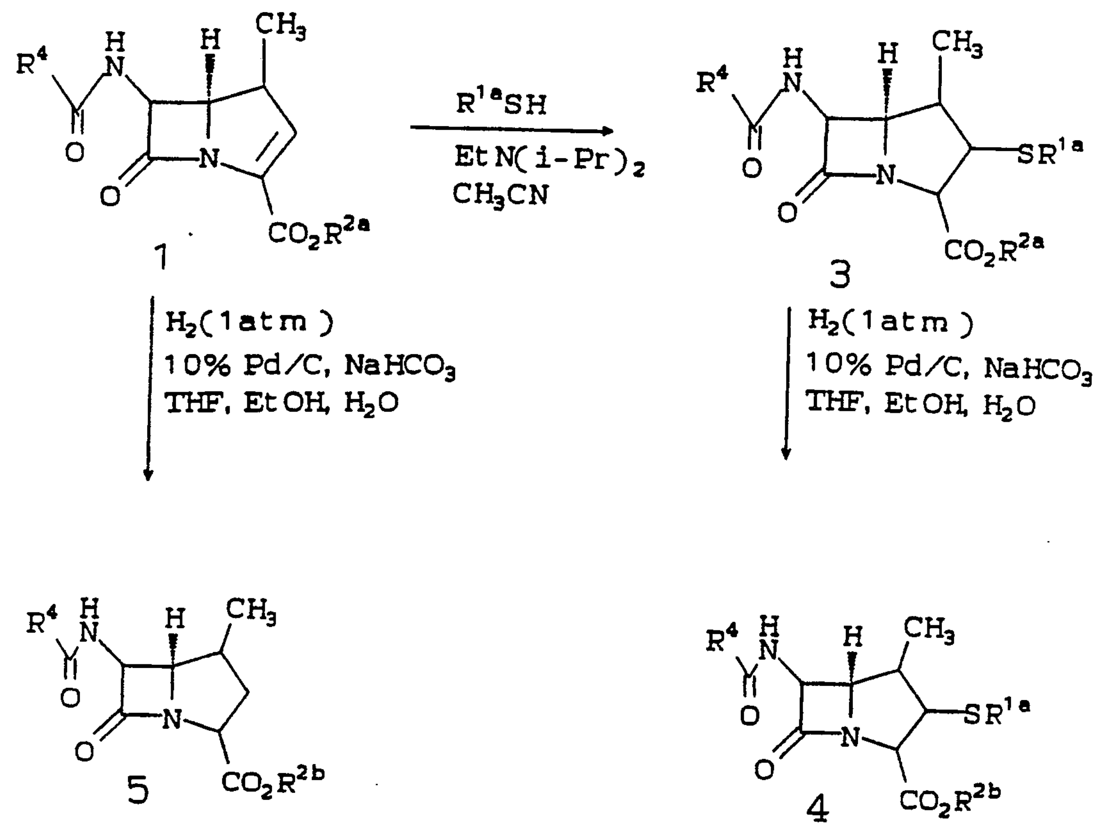


1 3 5 4

$SR^{1a}$ = $-SCH_3$, $-SC_6H_5$ or $S(CH_2)_nCN$ where n is 1 to 6 and preferably 2.
$R^{2a}$ = protecting group. i.e., allyl, p-nitrobenzyl, or benzyl
$R^{2b}$ = pharmaceutically acceptable salt or biolabile ester

$R^4$=

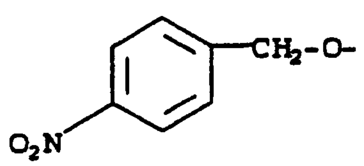

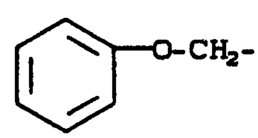

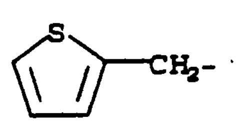

or

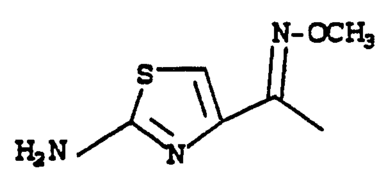

Flow Sheet A illustrates the synthesis of the carbapenams 4, 5 and 7 of the instant invention through intermediates 2, 3 and 6 from starting materials 1a and 1. Of most interest herein is the synthesis proceeding from starting material 1a through intermediates 2 and 3. This synthesis provides the greatest versatility in end product composition beginning conveniently with a single starting material, i.e. starting material 1a.

Starting materials 1a and 1 are known in the art from U.S Serial No. 213,398, filed June 30, 1988, corresponding to EP 350210. The synthesis of starting material 1a is illustrated in Flow Sheet B from well known reactants.

## Flow Sheet B

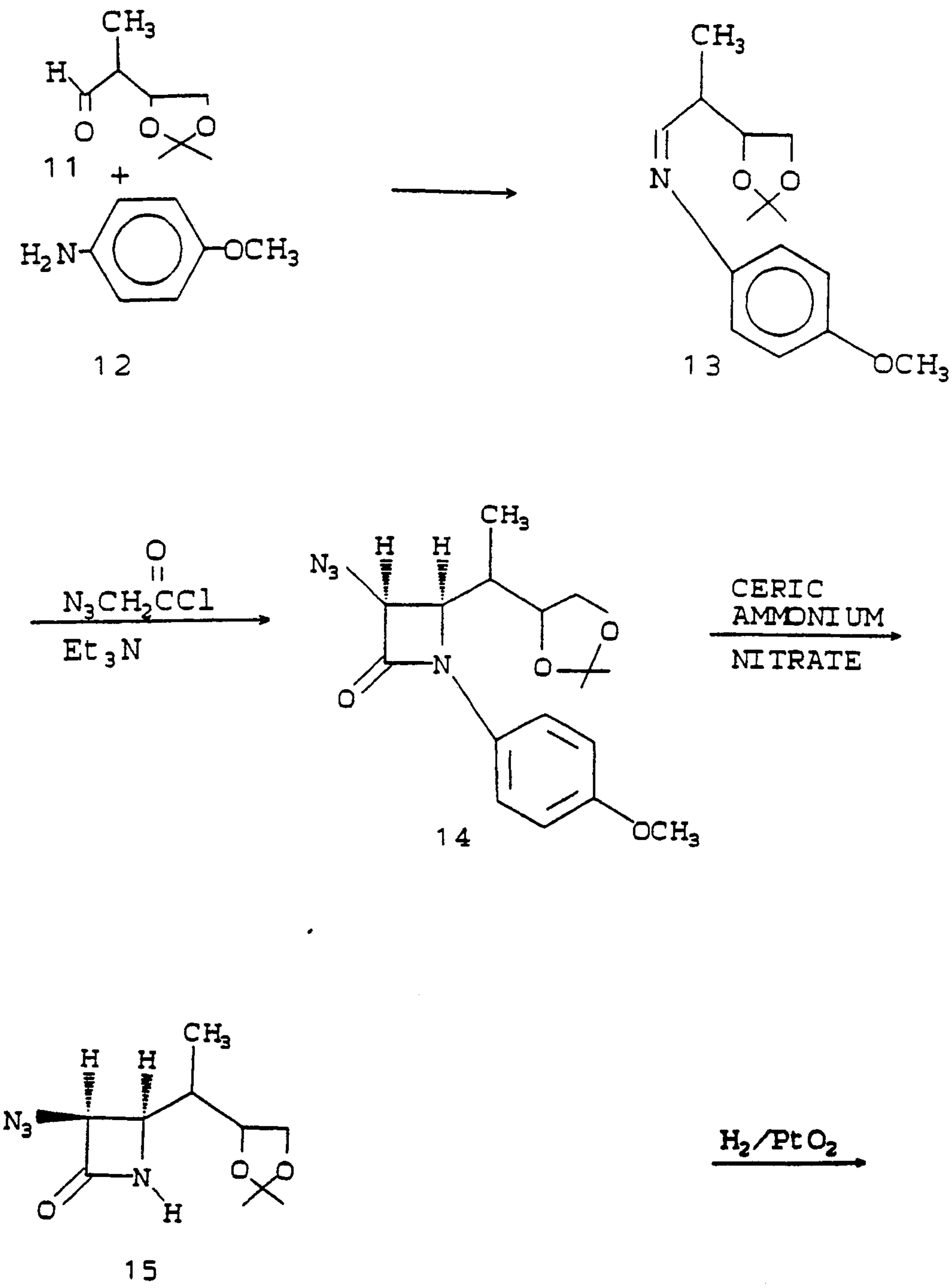


11 + 12 → 13 → 14 → 15

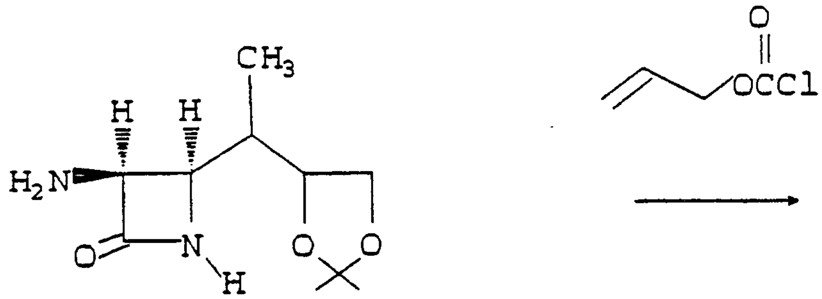

16

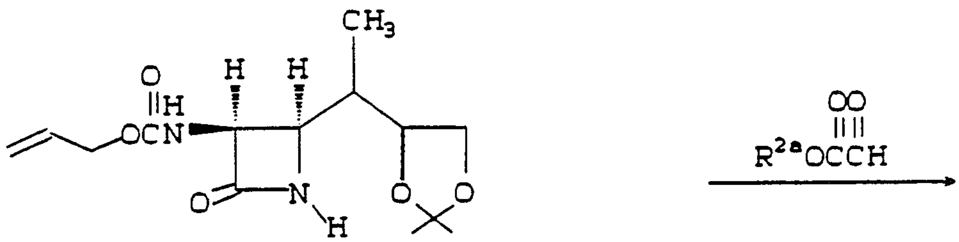

17

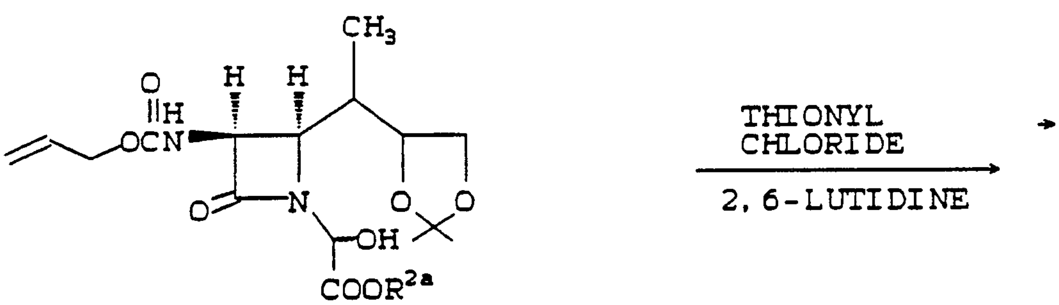


18

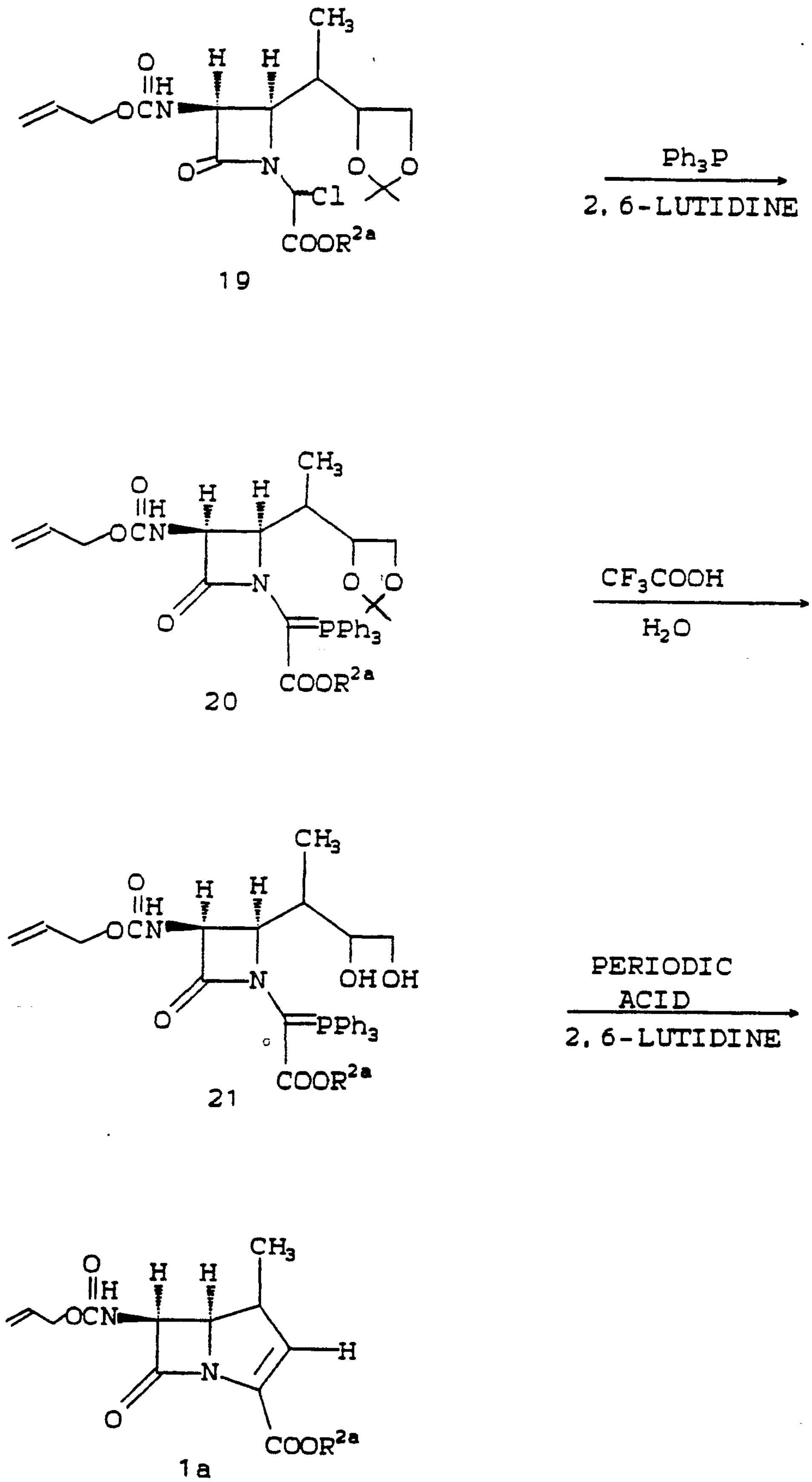


19

20

21

1a

To produce starting material 1a, the known starting aldehyde 11 is reacted with a suitable amine, e.g. aniline, benzylamine, 2,4-dimethoxybenzylamine, and preferably p-methoxyaniline 12, to produce the imine 13.

The imine 13, can be reacted with azidoacetyl chloride in the presence of a tertiary amine such as triethylamine or diisopropylethylamine or the like to produce the azetidinone 14.

Treating azetidinone 14 with ceric ammonium nitrate at 0 °C in acetonitrile to deblock the ring amide nitrogen yields compound 15. Catalytic hydrogenation of the azido group using $PtO_2$ yields the amino derivative 16.

Acylation to provide an allyloxycarbonyl derivative can be carried out with allyl chloroformate in anhydrous $CH_2Cl_2$ at 0 °C under nitrogen with pyridine as a proton acceptor to yield compound 17. Alternatively, at this point, an intermediate for the synthesis of starting material 1 could be produced by acylation of 16 with $R^4(C=O)Cl$. Reaction of 17 with allyl glyoxalate in anhydrous methylene chloride with 4A molecular sieves yields compound 18. Treating 18 with thionyl chloride and 2,6-lutidine in anhydrous THF at -40 °C yields the chloro analog 19 and contacting the chloro compound 19 in situ with triphenylphosphine/2,6-lutidine in anhydrous DMF at 80 °C for three hours yields the triphenylphosphorane 20. Hydrolysis of 20 with trifluoroacetic acid/water at 20 °C yields the dihydroxy compound 21. Oxidation of 21 in anhydrous methylene chloride at 45-47 °C for 15-30 minutes, with periodic acid/2,6-lutidine proceeds with concommitant cyclization of the resulting aldehyde to afford carbapenem 1a.

Suitable $R^1$, $R^3$ and $R^4$ in structure are I are well defined above. $R^2$, including $R^{2a}$ and $R^{2b}$, are not critical in terms of particular chemical identity but are more properly defined in terms of well known functions. $R^2$ as a protecting group is designated $R^{2a}$. Suitable $R^{2a}$ are well known and might vary depending not only on the reaction from which the carboxylic acid is being protected but also on the intended method for removing the protecting group. Shown in Flow Sheet A the protecting group is removed by hydrogenation. Thus, $R^{2a}$ is preferably benzyl or p-nitrobenzyl. Alternatively, $R^{2a}$ might be removed by oxidation, in which case, $R^{2a}$ might be p-methoxybenzyl or p-methoxyphenyl. Persons skilled in the art will readily understand that the removal of the protecting group $R^{2a}$ should not adversely effect the 6 position substitution on the carbapenam, i.e. $R^4(C=0)N$- or allyloxycarbonylamino. $R^2$ as hydrogen, a pharmaceutically acceptable salt or biolabile ester group is designated $R^{2b}$. Suitable $R^{2b}$ as pharmaceutically acceptable salts include Na or K. Suitable $R^{2b}$ as biolabile esters are metabolizable esters known in the penicillin, cephalosporin and penem arts to be easily cleaved within the body to the parent acid. Examples of such esters are indanyl, phthalidyl, methoxymethyl, glycyloxymethyl, phenylglycyloxy- methyl, thienylglycyloxymethyl, acetoxymethyl and pivaloyloxymethyl.

Referring to Flow Sheet A, the starting material 1a is reacted with a suitable substituted thiol in the presence of a base in a Michael -type addition to produce intermediate 2, a 2-(substituted-thio)carbapenam (see Example 1). The substituted thiol is $R^{1a}SH$ where $R^{1a}$ is defined above. Preferred as a base for the above reaction is a trialkylamine such as diisopropylethylamine or triethylamine. Other bases which might be employed include tetra-n-butylammonium fluoride, 1,8-diazabicyclo[5.4.0]undec-7-ene and potassium carbonate. The reaction is carried-out in a polar organic solvent such as acetonitrile at moderate temperatures, generally between -30 °C and 20 °C. Other solvents which are equally acceptable included tetrahydrofuran and N,N-dimethylformamide. Reaction of starting material 1a with a thiol $R^{1a}SH$ produces compound 2 as an isomeric mixture (see Example 1). These isomers are separable by chromatography on silica gel and are individually converted into final products 4 and 7.

The amino function of compound 2 is deprotected in a conventional manner by a palladium (0) catalyzed deallylation-decarboxylation reaction and the resulting amine is acylated in situ to produce compound 3 (see Examples 2 and 3). Acylation of the amine intermediate can be carried-out with $R^4(C=0)$-Cl, where $R^4$ is as defined above, and pyridine as a proton acceptor for from 0.5 to 6 hours at room temperature to yield compound 3.

The carboxyl group of compound 3 is deprotected by hydrogenolysis of $R^{2a}$ to yield a subject product 4 where $R^{2b}$ is hydrogen or, when the reaction is carried-out in the presence of an appropriate alkali metal hydrogen carbonate a subject product 4 where $R^{2b}$ is a pharmaceutically acceptable salt. Deprotection may also be carried out in such a manner as to result in a biolabile ester as $R^{2b}$.

Alternatively, compound 2 can be deprotected to the corresponding amine as described above which can be reacted with 1-(chloromethylene)-hexahydro-1H-azepinium chloride and triethylamine in anhydrous $CH_2Cl_2$ to yield amidino carbapenam 6 (see Example 4). As with compound 3, the carboxyl of compound 6 can be deprotected to yield product 7.

Compound 1 may be employed in two alternative syntheses to produce product compounds. Compound 1 may be directly hydrogenated both saturating the double bond and deprotecting the carboxyl

group in a single step to produce product compound 5 (see Example 6). Otherwise, compound 1 may be reacted with a suitable substituted thiol in a Michael type addition as described above to produce intermediate 3.

The novel compounds in the different chemical classes of the present disclosure are believed to be valuable antibiotics active against various Gram-positive and Gram-negative bacteria and accordingly find utility in human and veterinary medicine. Representative pathogens which are sensitive to antibiotics I include: Staphylococcus aureus, and Escherichia coli. The antibacterials of the invention are not limited to utility as medicaments; they may be used in all manner of industry, for example: additives to animal feed, perservation of food, disinfectants and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The products of this invention may be used in any of a variety of pharmaceutical preparations. They may be employed in capsule, powder form, in liquid solution, or in suspension. They may be administered by a variety of means; those of principal interest include: orally, topically or parenterally by injection (intravenously or intramuscularly).

Such tablets and capsules, designed for oral administration, may be in unit dosage form, and may contain conventional excipients, such as binding agents, for example, syrup, acacia, gelatin, sorbitol, tragacanth, or polyvinylpyrrolidone; fillers, for example, lactose, sugar, cornstarch, calcium phosphate, sorbitol, or glycerine; lubricants, for example, magnesium stearate, talc, polyethylene glycol, silica; disintegrants, for example, potato starch, acceptable wetting agents such as sodium lauryl sulphate. The tablets may be coated according to methods well known in the art. Oral liquid preparations may be in the form of aqueous or oily suspensions, or solutions, or they may be presented as a dry product for reconstitution with water or other suitable vehicle before use. Such liquid preparations may contain conventional additives such as suspending agents, for example, sorbitol, methyl cellulose, glucose/sugar syrup, gelatin, hydroxyethylcellulose, or carboxymethyl cellulose. Suppositories will contain conventional suppository bases, such as cocoa butter or other glycerides.

Compositions for injection, the preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water.

The compositions may also be prepared in suitable forms for absorption through the mucous membranes of the nose and throat or bronchial tissues and may conveniently take the form of liquid sprays or inhalants, lozenges, or throat paints. For medication of the eyes or ears, the preparation may be presented in liquid or semi-solid form. Topical applications may be formulated in hydrophobic or hydrophilic bases as ointments, creams, lotions, paints, or powders.

The dosage to be administered depends to a large extent upon the condition and size of the subject being treated as well as the route and frequency of administration--the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg. of body weight of the subject in one or more treatments per day. A preferred daily dosage for adult humans lies in the range of from about 10 to 240 mg. of active ingredient per kg. of body weight. Another factor influencing the precise dosage regimen, apart from the nature of the infection and peculiar identity of the individual being treated, is the molecular weight of the chosen species of this invention.

The compositions for human delivery per unit dosage, whether liquid or solid, may contain from 0.1% to 99% of active material, the preferred range being from about 10-60%. The composition will generally contain from about 15 mg. to about 1500 mg. of the active ingredient; however, in general, it is preferable to employ a dosage amount in the range of from about 250 mg to 1000 mg. In parenteral administration, the unit dosage amount is usually the pure compound I in sterile water solution or in the form of a soluble powder intended for solution.

The following examples are illustrative of the invention and should not be construed as being limits on the scope and spirit of the instant invention.

## EXAMPLE 1

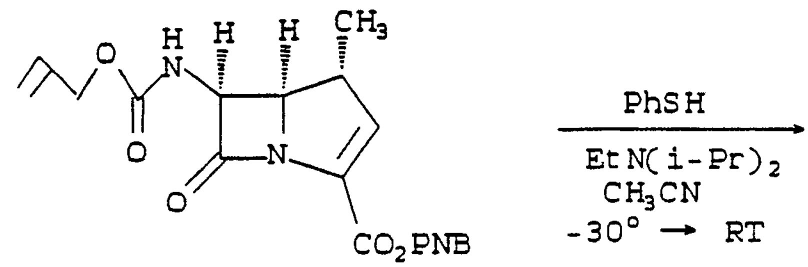


E1

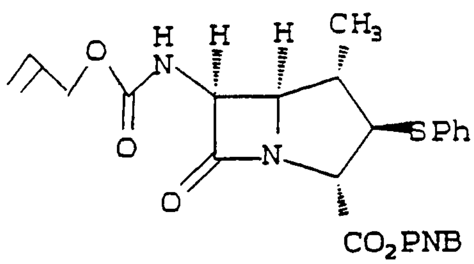

+

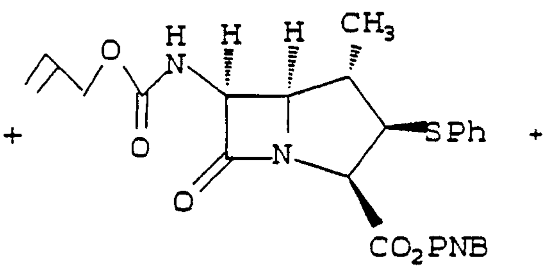

+

E2 E3

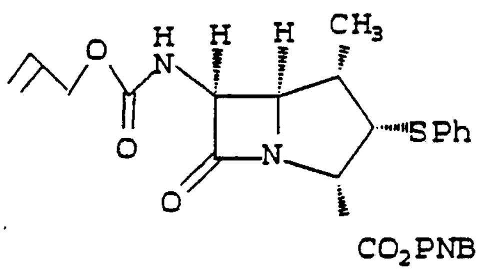

E4

(±)-p-Nitrobenzyl-(1S,2R,3S,5R,6S,)-6-allyloxycarbonylamino-1-methyl-2-phenylthio-carbapenam-3-carboxylate (E2), (±)-p-Nitrobenzyl-(1S,2R,3R, 5R,6S)-6-allyloxycarbonylamino-1-methyl-2-phenylthio-carbapenam-3-carboxylate (E3), and (±)-p-Nitrobenzyl-(1S,2S,3S,5R,6S)-6-allyloxycarbonylamino-1-methyl-2-phenylthio-carbapenam-3-carboxylate (E4)

To a solution of the carbapenem E1 (39.0 mg, 0.0973 mmol) in 0.5 ml of acetonitrile at -30°C was added thiophenol (0.011 ml, 0.10 mmol) followed by a solution of diisopropylethylamine in acetonitrile (0.19 M, 0.10 ml). The reaction mixture was allowed to warm to room temperature during 2 hours, and was then diluted with ethyl ether and washed with pH 7 phosphate buffer and brine. Drying ($MgSO_4$) and evaporation gave an oil which was separated by preparative TLC on silica gel (1:1 EtOAC/hexane) to yield 11.8 mg

(23.7%) of the fastest eluting isomer, carbapenam E2, and 19.2 mg of an oil which contained the remaining isomers. Further separation of this oil by preparative TLC on silica gel (7:2:1 $CH_2Cl_2$/hexane/EtOAC) yielded 9.2 mg (19%) of carbapenam E3 and 3.9 mg (7.8%) of the more polar carbapenam E4.

Carbapenam E2

$^1$H-NMR (300 MHz, $CDCl_3$): δ 1.21 (d, J=6.59 Hz, $CH_3$), 1.75-1.90 (m, H1), 3.65-3.80 (m, 2H, H2 and H5), 4.33 (d, J=4.33 Hz, H3), 4.59 (d, J=4.64 Hz, -$OCH_2C$=C), 5.05-5.45 (m, 5H, H6, -$OCH_2$Ar, -C=$CH_2$), 5.85-6.0 (m, -CH=C), 7.25-7.50 (m, 7H, ArH), 8.20 (d, J=8.79 Hz, 2H, ArH).

IR($CRCl_3$): 3435 (NH), 1780 (β-lactam), 1745 (ester), 1730 $cm^{-1}$ (carbamate).

FAB-MS: m/e = 512 (M+H).

Carbapenam E3

$^1$H-NMR (300 MHz, $CDCl_3$): δ 1.21 (d, J=6.78 Hz, $CH_3$), 2.15-2.35 (m, H1), 3.58 (dd, J=4.3, 9.1 Hz, H5), 3.62 (dd, J=7.69, 12.1 Hz, H2), 4.38 (d, J=7.69 Hz, H3), 4.58 (d, J=6.3 Hz, -$OCH_2C$=C), 5.07 (dd, J=4.3, 7.6 Hz, H6), 5.17 (br d, J=7.6 Hz, NH), 5.20-5.45 (m, 2H, -C=$CH_2$), 5.31 (d, J=4.4, -$OCH_2$Ar), 5.80-5.95 (m, 1H, CR=$CH_2$), 7.20-7.35 (m, 5H, -SPh), 7.49 (d, J=8.85, 2H, PNB ArH), 8.20 (d, J=8.85, 2H, PNB ArH).

IR ($CRCl_3$): 3430 (NH), 1780 (β-lactam), 1735 (ester), 1725 $cm^{-1}$ (carbamate).

FAB-MS: m/e = 512 (M+H).

Carbapenam E4

$^1$H-NMR (300 MHz, $CDCl_3$): δ 1.22(d, J=6.47 Hz, $CH_3$), 2.20-2.35 (m, H1), 3.92 (dd, J=4.5, 9.0 Hz, H5), 4.23 (t, J=6.5 Hz, H2), 4.59 (d, J=4.8 Hz, -$OCH_2C$=C) 4.80 (d, J=6.5 Hz, H3), 5.12 (d, J=13.3 Hz, -$OCH_AAr$), 5.15-5.35 (m, 4H, H6, -C=$CH_2$, NH), 5.25 (d, J=13.3 Hz, -$OCH_BAr$), 5.85-6.0 (m, -CH=C), 7.20-7.35 (m, 5H, -SPh), 7.41 (d, J=8.12 Hz, 2H, PNB ArH), 8.13 (d, J=8.12 Hz, 2H, PNB ArH).

IR ($CRCl_3$): 3440 (NH), 1775 (β-lactam), 1730 $cm^{-1}$ (ester, carbamate)

FAB-MS: m/e = 512 (M+H).

EXAMPLE 2

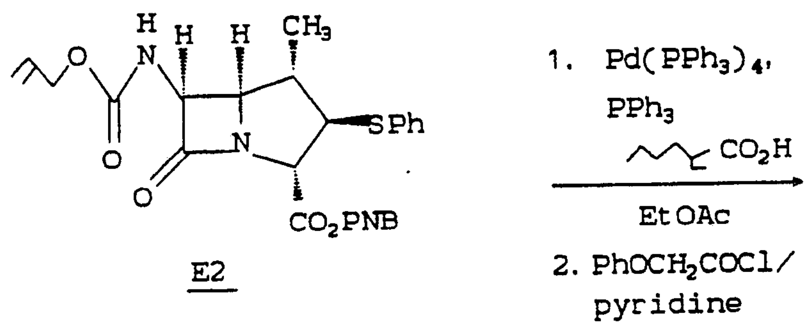


E2

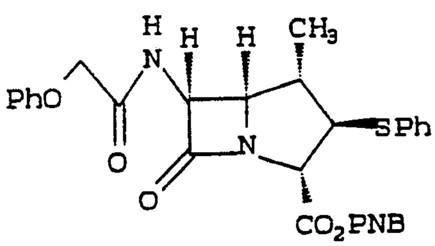

E5

(±)-p-Nitrobenzyl-(1S,2R,3S,5R,6S)-1-methyl-6-phenoxyacetamido-2-phenylthio-carbapenam-3-carboxylate

(E5)

To a solution of the carbapenam E2 (10.0 mg, 0.0196 mmol) in 0.3 ml of ethyl acetate were added in sequence triphenylphosphine (0.050 ml of a 0.12 M solution in EtOAC), tetrakis(triphenylphosphine)-palladium (0.050 ml of a 0.020 M solution in $CH_2Cl_2$), and 2-ethylhexanoic acid (0.0090 ml, 0.059 mmol). After stirring at room temperature for 1 hour, pyridine (0.006 ml, 0.08 mmol) was added followed by phenoxyacetyl chloride (0.004 ml, 0.03 mmol). After an additional 1 hour, the reaction mixture was diluted with ethyl ether and washed with pH 7 phosphate buffer and brine. Drying ($MgSO_4$) and evaporation left an oil which was purified by preparative TLC on silica gel (1:1 EtOAC/hexane) to yield 6.7 mg (61%) of the title compound as a yellow oil.

$^1$H-NMR (200 MHz, $CDCl_3$): $\delta$ 1.15 (d, J=6.6 Hz, $CH_3$), 1.6-1.8 (m, H1), 3.67 (dd, J=7.5, 9.7 Hz, H2), 3.80 (dd, J=4.7, 7.9 Hz, H5), 4.35 (d, J=7.5 Hz, $H_3$), 4.55 (s, -$CH_2$OPh), 5.10-5.25 (AB, -$CH_2$OAr), 5.40 (dd, J=4.7, 6.8 Hz, H6), 6.96 (d, J=7.6 Hz, 2H, ArH), 7.11 (t, J=8 Hz, 1H, ArH), 7.2-7.5 (m, 9H, ArH), 8.24 (d, J=8.9 Hz, 2H, ArH).

IR($CHCl_3$): 3420 (NH), 1780 ($\beta$-lactam), 1745 (ester), 1690 cm$^{-1}$ (amide)

FAB-MS: m/e = 562 (M+H)

## EXAMPLE 3

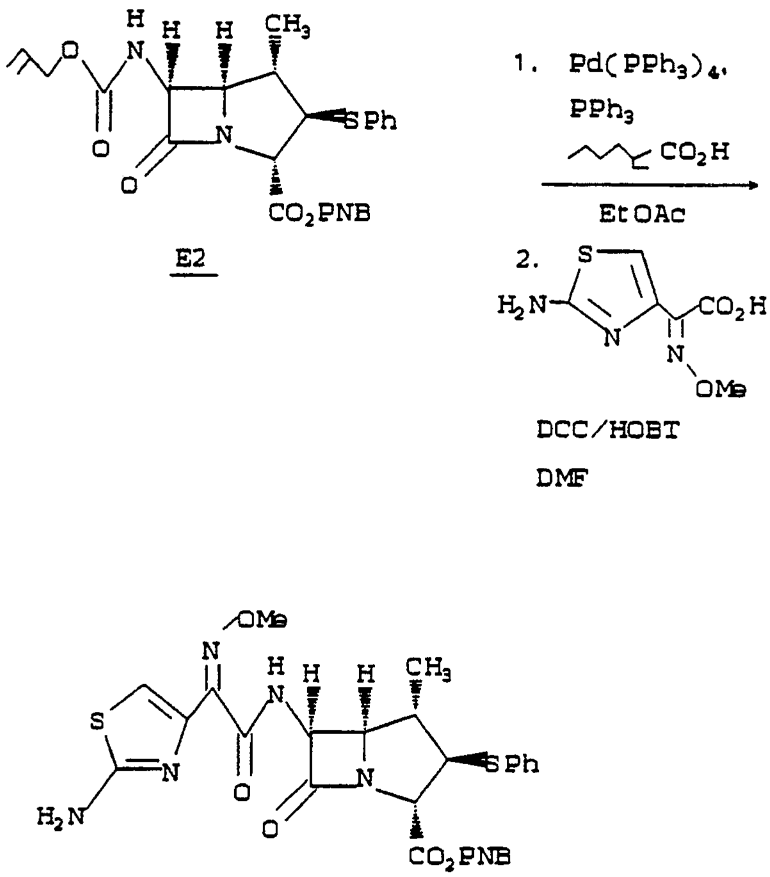


E2

E6

(±)-p-Nitrobenzyl-(1S,2R,3S,5R,6S)-6-[(2-amino-4-thiazolyl)methoxyiminoacetamido]-1-methyl-2-phenylthio-carbapenam-3-carboxylate (E6)

To a solution of the carbapenam E2 (7.0 mg, 0.014 mmol) in 0.5 ml of ethyl acetate were added triphenylphosphine (0.050 ml of a 0.082 M solution in EtOAC, 0.0041 mmol), tetrakis(triphenylphosphine)-palladium (0.050 ml of a 0.027 M solution in $CH_2Cl_2$, 0.0014 mmol), and 2-ethylhexanoic acid (0.0065 ml, 0.041 mmol). After stirring at room temperature for 1 hour, a mixture of (2-amino-4-thiazolyl)-methoxyiminoacetic acid (3.6 mg, 0.018 mmol), 1,3-dicyclohexylcarbodiimide (3.8 mg, 0.018 mmol), and 1-hydroxybenzotriazole hydrate (2.9 mg, 0.018 mmol) in 0.5 ml of N,N-dimethylformamide (which had been stirred at room temperature for 1 hour) was added. After stirring at room temperature for an additional 2 hours, the reaction mixture was diluted with ethyl acetate and washed with sat. $NaHCO_3$, water, and brine. Drying ($MgSO_4$), evaporation, and separation by preparative TLC on silica gel (1:1 EtOAc/$CH_2Cl_2$) yielded 3.2 mg (38%) of the title compound as an oil.

$^1$H-NMR (300 MHz, $CDCl_3$): $\delta$ 1.24 (d, J=6.53 Hz, $CH_3$), 1.85-2.00 (m, H1), 3.71 (dd, J=6.9, 10.1 Hz, H2), 3.86 (dd, J=4.7, 7.8 Hz, H5), 4.08 (s, $OCH_3$), 4.38 (d, J=6.9 Hz, H3), 5.11 (d, J=7.0 Hz, $OCH_A$-Ar), 5.19 (d, J=7.0 Hz, -$OCH_B$-Ar), 5.42 (dd, J=4.7, 7.0 Hz, H6), 6.95 (s, 1H, thiazole-H), 7.20-8.25 (m, 9H, ArH).

IR($CRCl_3$): 1780 ($\beta$-lactam), 1745 (ester), 1680 cm$^{-1}$ (amide)

## EXAMPLE 4

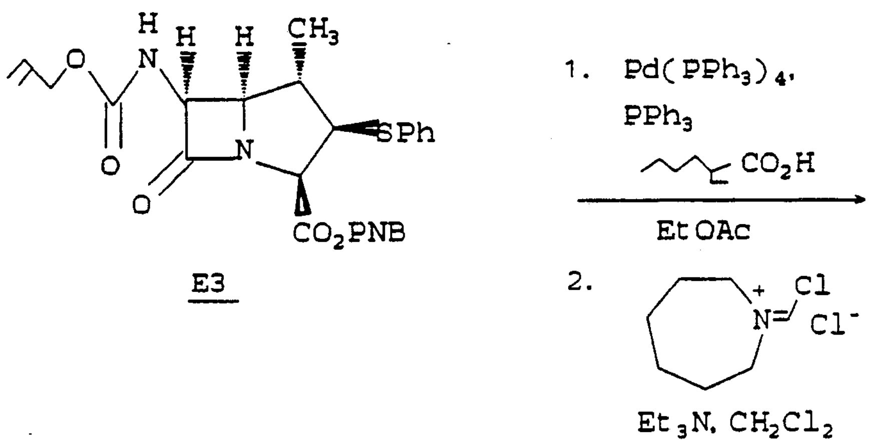


E3

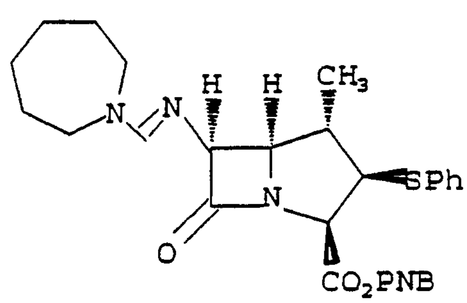

E7

(±)-p-Nitrobenzyl-(1S,2R,3R,5R,6S)-6-[(hexahydro-1H-azepin-1-yl)methyleneamino]-1-methyl-2-phenylthio-carbapenam-3-carboxylate (E7)

To a solution of the carbapenam E3 (14.0 mg, 0.0274 mmol) in 0.5 ml of ethyl acetate were added in sequence triphenylphosphine (0.050 ml of a 0.16 M solution in EtOAC, 0.008 mmol), tetrakis-(triphenylphosphine)palladium (0.10 ml of a 0.027 M solution in $CH_2Cl_2$, 0.0027 mmol), and 2-ethylhexanoic acid (0.013 ml, 0.082 mmol). After 1 hour, triethylamine (0.015 ml, 0.11 mmol) was added followed by a solution of 1-(chloromethylene)-hexahydro-1H-azepinium chloride in methylene chloride [prepared by the addition of oxalyl chloride (0.0036 ml, 0.041 mmol) to a solution of N-formylhexahydro-1H-azepine (5.2 mg, 0.041 mmol) in 0.4 ml of methylene chloride]. After stirring for 3 hours more at room temperature, the reaction mixture was diluted with ethyl ether and washed with pH 7 phosphate buffer, sat. $NH_4Cl$, and brine. Drying ($MgSO_4$), evaporation under reduced pressure, and purification by preparative TLC on silica gel (1:1 $EtOAC/CH_2Cl_2$) yielded 7.0 mg (46%) of the title compound as an oil.
$^1H$-NMR (300 MHz, $CDCl_3$): $\delta$ 1.19 (d, J=6.59 Hz, $CH_3$), 1.5-1.8 (m, 8H, $-CH_2CH_2CH_2CH_2-$), 2.45-2.60 (m, H1), 3.20-3.35 (m, 4H, $-CH_2NCH_2-$), 3.44 (dd, J=4.4, 8.9 Hz, H5), 3.55 (dd, J=7.7, 12.0 Hz, H2), 4.39 (d, J=7.7 Hz, H3), 4.78 (d, J=4.4 Hz, H6,), 5.25-5.40 (AB, $-OCH_2-$), 7.20-7.75 (m, 7H, ArH), 7.61 (s, 1H, -N=CH-), 8.16 (d, J=8.9 Hz, 2H, ArH).
IR($CHCl_3$): 1765 ($\beta$-lactam), 1745 (ester), 1635 $cm^{-1}$ (imine).

EXAMPLE 5

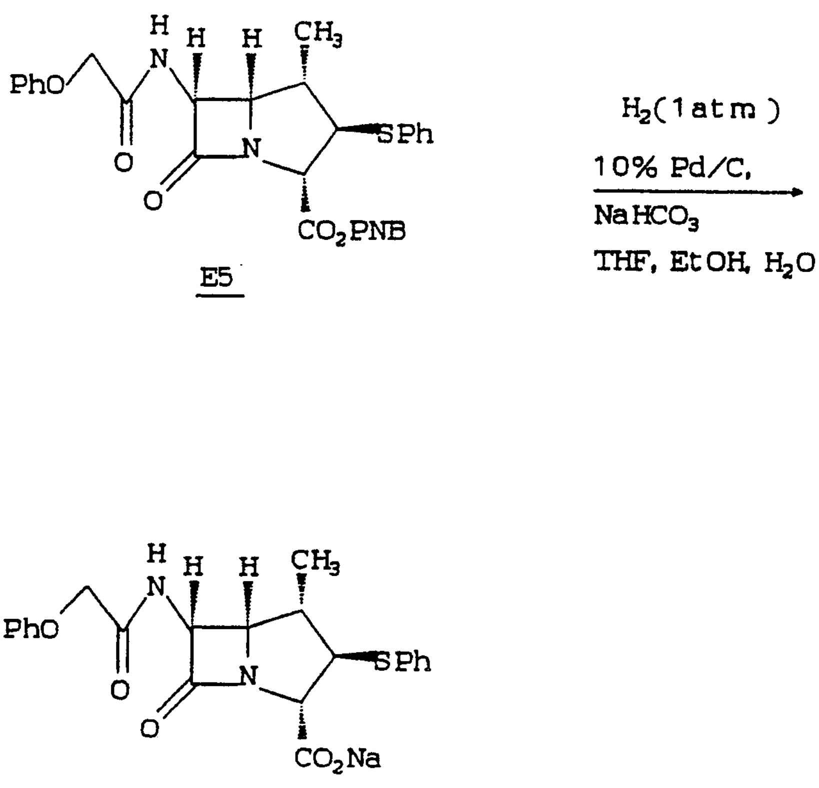


E5

E8

(±)-Sodium-(1S,2R,3S,5R,6S)-1-methyl-6-phenoxyacetamido-2-phenylthio-carbapenam-3-carboxylate (E8)

To a solution of the carbapenam E5 (6.5 mg, 0.012 mmol) in tetrahydrofuran (0.2 ml) - ethanol (0.2 ml) - water (0.4 ml) were added sodium bicarbonate (0.025 ml of a 0.48 M solution in $H_2O$, 0.012 mmol) and 10% palladium on carbon (6.5 mg). The reaction mixture was hydrogenated at atmospheric pressure for 3 hours, and was then filtered through celite, washing the filter pad with water. The filtrate was extracted with ethyl acetate and the aqueous phase was lyophilized to give an off-white solid. Purification by reverse-phase preparative TLC (4:1 $H_2O/THF$) yielded 2.1 mg (40%) of the title compound as a white lyophilized solid.

$^1$H-NMR (300 MHz, $D_2O$): δ 0.95 (d, J=6.59 Hz, $CH_3$), 1.55-1.75 (m, H1), 3.60 (dd, J=6.9, 8.7 Hz, H2), 3.74 (dd, J=4.5, 8.5 Hz, H5), 4.05 (d, J=6.9 Hz, $H_3$), 4.69 (s, -$CH_2$OPh), 5.26 (d, J=4.5 Hz, H6), 6.95-7.60 (m, 10H, ArH).

EXAMPLE 6

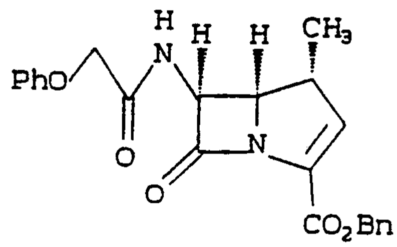

E9

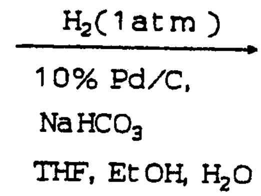


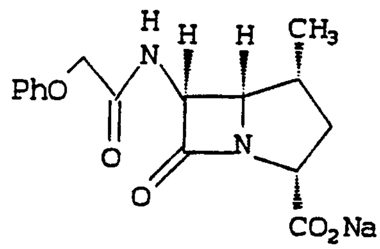

E10

(±)-Sodium-(1R,3R,5R,6S,)-1-methyl-6-phenoxyacetamido-carbapenam-3-carboxylate (E10)

In a manner analogous to that described in Example 5, the carbapenem E9 (10.0 mg, 0.0242 mmol) was hydrogenated to yield the title compound (4.8 mg, 59%) as an off-white lyophilized solid.
$^1$H-NMR (300 MHz, $D_2O$): δ 0.93 (d, J=5.18 Hz, $CH_3$), 2.05-2.40 (m, 3H, H1, H2), 3.60 (dd, J=4.5, 8.9 Hz, H5), 3.94 (d, J=8.1 Hz, H3), 4.81 (S, -$CH_2$OPh), 5.21 (br d, J=4.5 Hz, H6), 7.09 (d, J=8.9 Hz, 2H, ArH), 7.14 (t, J=7.2 Hz, 1H, ArH), 7.45 (dd, J=7.2, 8.9 Hz, 2H, ArH).

EXAMPLES 7-16

Operating as described in the previous examples, the following compounds were analogously prepared:

$^1$H-NMR(300 MHz, $D_2O$)

| | Ex No. | H2 | H3 | H5 | H6 |
|---|---|---|---|---|---|
| 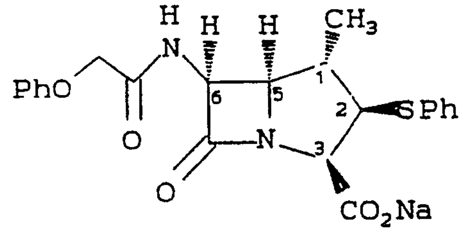 | 7 | δ3.75,dd J=8.5, 11.7Hz | 4.19,d J=8.5 | 3.63,dd J=4.5, 9.2 | 5.22,d J=4.5 |
| 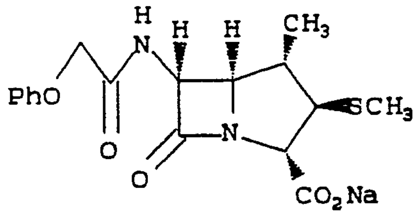 | 8 | 3.1,dd J=8.2, 11.4 | 4.03,d J=8.2 | 3.70,dd J=4.7, 8.3 | 5.27,d J=4.7 |
| 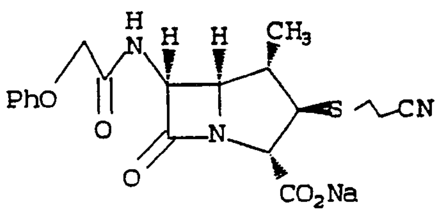 | 9 | 3.24,dd J=8.2, 10.1 | 4.07,d J=8.2 | 3.73,dd J=4.5, 9.3 | 5.31,d J=4.5 |

| | Ex No. | H2 | H3 | H5 | H6 |
|---|---|---|---|---|---|
| 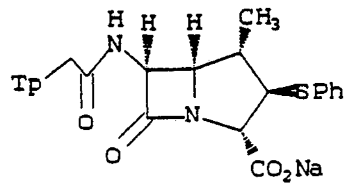 | 10 | 3.56,dd J=7.8, 9.3 | 4.07,d J=7.8 | 3.73,dd J=4.3, 8.3 | 5.16,d J=4.3 |
| 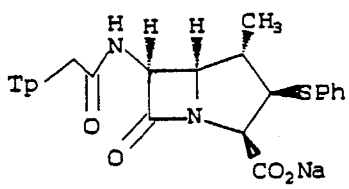 | 11 | 3.74,dd J=8.4, 12.6 | 4.17,d J=8.4 | 3.60,dd J=4.5, 9.3 | 5.10,d J=4.5 |
| 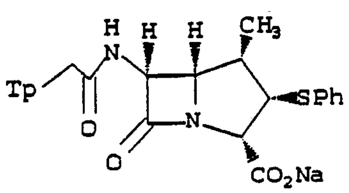 | 12 | 4.30,t J=6.2 | 4.60,d J=6.2 | 3.8,dd partially obscured | 5.26,d J=4.3 |

| | Ex. No. | H2 | H3 | H5 | H6 |
|---|---|---|---|---|---|
| 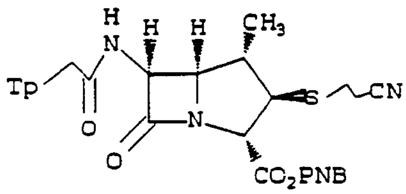 | 13 | 3.25,dd J=7.5, 10.0 | 4.08,dd J=7.5 | 3.71,dd J=4.2, 8.7 | 5.23,d J=4.2 |
| 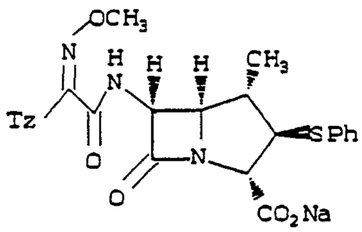 | 14 | 3.68,br t J=8 | 4.16,d J=8 | 3.90,dd J=4.3, 8.2 | 5.37,d J=4.3 |

| | Ex No. | H2 | H3 | H5 | H6 |
|---|---|---|---|---|---|
| 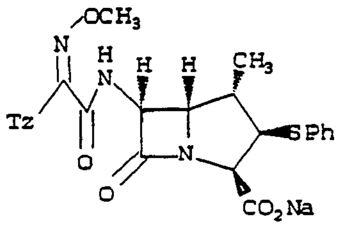 | 15 | 3.85,d<br>J=8.0, 10.9 | 4.24,d<br>J=8.0 | 3.77,dd<br>J=4.2, 10.0 | 5.32,d<br>J=4.2 |
| 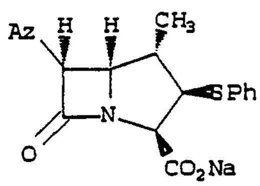 | 16 | 3.84,dd<br>J=8.3, 10.7 | 4.25,d<br>J=8.3 | 3.71,dd<br>J=4.4, 9.2 | 5.04,d<br>J=4.4 |

Ph= 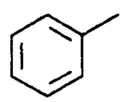 Az= 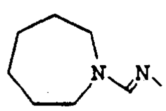 Tp= 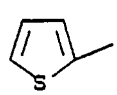 Tz= 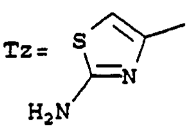

## Claims

1. A compound of the structural formula:

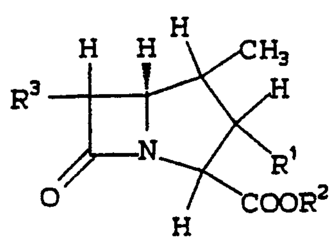

wherein
$R^1$ is -H or S-$R^{1a}$;
$R^{1a}$ is -$CH_3$,-$C_6H_5$ or -$(CH_2)n$ CN;
n is 1 to 6;
$R^2$ is hydrogen, protecting group, pharmaceutically acceptable salt, or biolabile ester group;

$R^3$ is

(a)

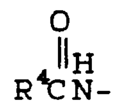

or

(b)

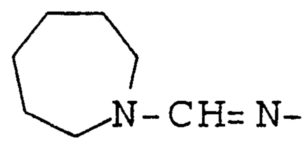 ; and

$R^4$ is (a) allyloxy;

(b) 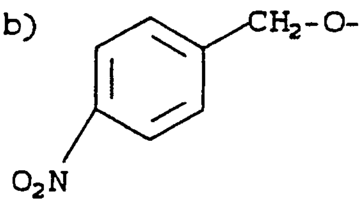 (c) 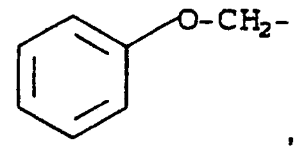 ,

(d) 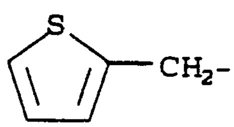 or (e) 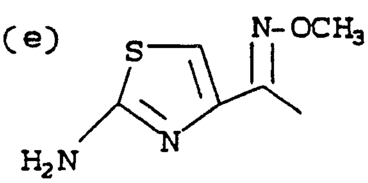 .

2. The compound of Claim 1 wherein n is 2.

3. The compound of Claim 1 wherein $R^2$ is hydrogen.

4. The compound of Claim 1 wherein $R^2$ is a protecting group selected from the group consisting of allyl, p-nitrobenzyl, benzyl, methoxybenzyl, and methoxyphenyl.

5. The compound of Claim 1 wherein $R^2$ is a pharmaceutically acceptable salt selected from the group consisting of Na and K.

6. The compound of Claim 1 wherein $R^3$ is

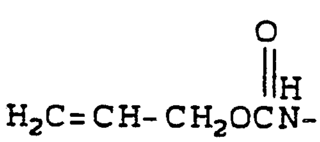 or 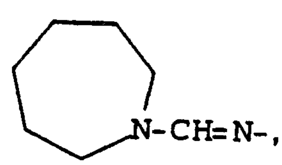

7. The compound of Claim 1 wherein $R^4$ is;

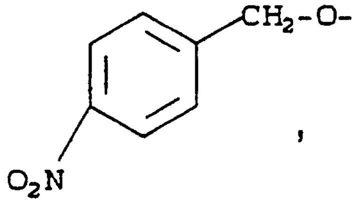 , 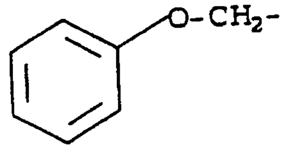 ,

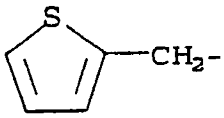 or 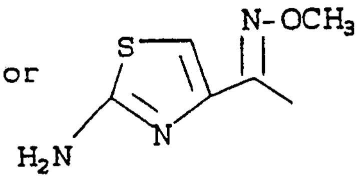 .

8. A compound of the structural formula;

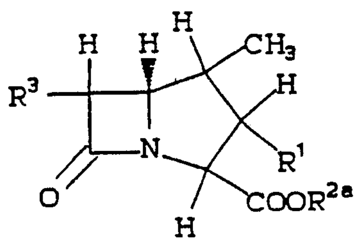

wherein
$R^1$ is -H or -S-$R^{1a}$;
$R^{1a}$ is $-CH_3$,$-C_6H_5$ or $-(CH_2)_nCN$;
n is 1 to 6;
$R^{2a}$ is a protecting group selected from the group consisting of allyl, p-nitrobenzyl, benzyl, methoxybenzyl, and methoxyphenyl;

$R^3$ is

(a)

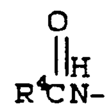

or

(b)

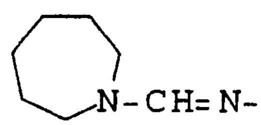 ; and

$R^4$ is (a) allyloxy;

(b) 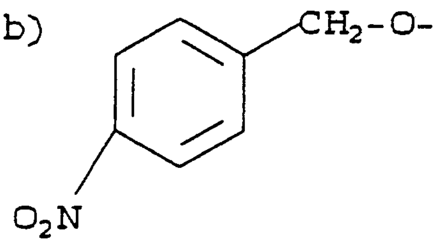 (c) 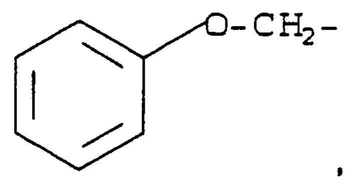 ,

(d) 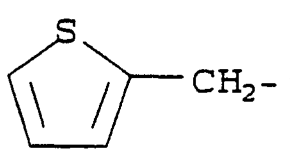 or (e) 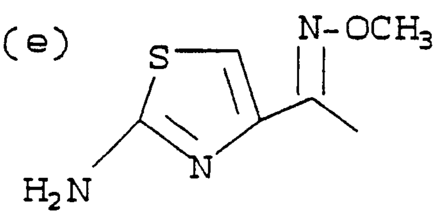 .

9. A compound of the structural formula;

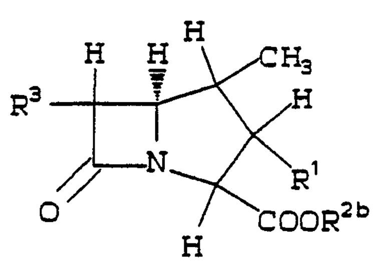

wherein

$R^1$ is -H or $-S-R^{1a}$;
$R^{1a}$ is $-CH_3$, $-C_6H_5$ or $-(CH_2)_nCN$;
n is 1 to 6; $R^{2b}$ is a pharmaceutically acceptable salt selected from K or Na, a biolabile ester or hydrogen;

$R^3$ is

(a)

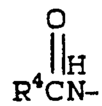

or

(b)

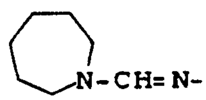 ; and

$R^4$ is (a) allyloxy:

(b) 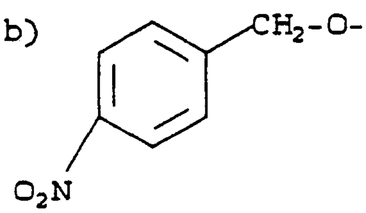 (c) 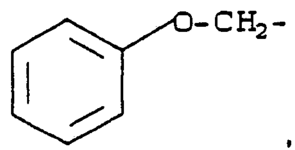 ,

(d) 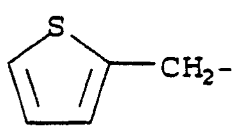 or (e) 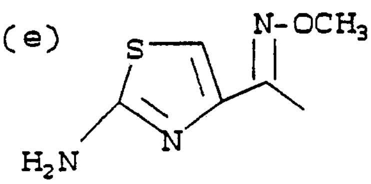 .

10. An antibacterial composition comprising a therapeutically effective amount of a compound of Claim 9 and a pharmaceutically acceptable carrier therefor.

European Patent Office

# EUROPEAN SEARCH REPORT

Application Number

EP 90 30 1145

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | US-A-4 347 355 (D.T. CHU)<br>* The whole document * | 1,9,10 | C 07 D 477/00<br>A 61 K 31/40 |
| Y | CHEMICAL ABSTRACTS, vol. 111, no. 11, 11th September 1989, page 715, abstract no. 96962z, Columbus, Ohio, US; & JP-A-63 310 890 (LEDERIE (JAPAN), LTD) 19-12-1988<br>* Abstract * | 1,9,10 | |
| D,P Y | EP-A-0 350 210 (MERCK)<br>* Claims 14,15,19,22,24 * | 1-10 | |

TECHNICAL FIELDS SEARCHED (Int. Cl.5)

C 07 D 477/00
A 61 K 31/00

The present search report has been drawn up for all claims

| Place of search | Date of completion of the search | Examiner |
|---|---|---|
| THE HAGUE | 12-04-1990 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document